# EUROPÄISCHE PATENTSCHRIFT

(11) **EP 3 056 227 B1**
(45) Veröffentlichungstag und Bekanntmachung des Hinweises auf die Patenterteilung: **20.09.2017**
(21) Anmeldenummer: 16152082.0
(22) Anmeldetag: 20.01.2016
(51) Int. Cl.: A61L 27/54, A61K 9/00, A61K 47/32, A61K 9/14, A61L 24/00, A61L 24/06

(54) **ANTIMYKOTISCHER POLYMERISIERBARER KNOCHENZEMENT UND EIN VERFAHREN ZU SEINER HERSTELLUNG**
ANTIMYCOTIC POLYMERISABLE BONE CEMENT AND A METHOD FOR THE PRODUCTION THEREOF
CIMENT OSSEUX POLYMERISABLE ANTIMYCOSIQUE ET SON PROCEDE DE FABRICATION

(30) Priorität: 16.02.2015 DE 102015102210
(43) Veröffentlichungstag der Anmeldung: 17.08.2016
(73) Patentinhaber: Heraeus Medical GmbH, 61273 Wehrheim (DE)
(72) Erfinder: Vogt, Sebastian, 99092 Erfurt (DE)
(74) Vertreter: Heraeus IP

(56) Entgegenhaltungen:
- EP-A2- 2 730 296
- DE-A1- 2 417 993
- DATABASE WPI Week 201016 Thomson Scientific, London, GB; AN 2010-B81401 XP002759229, & JP 2010 037252 A (MEDREX KK) 18. Februar 2010 (2010-02-18) -& DATABASE EPODOC [Online] EUROPEAN PATENT OFFICE, THE HAGUE, NL; XP002759230, Database accession no. jp-2008200727-a -& JP 2010 037252 A (MEDOREKKUSU KK) 18. Februar 2010 (2010-02-18)
- CLAUDIA SALERNO ET AL: "Lipid-based microtubes for topical delivery of Amphotericin B", COLLOIDS AND SURFACES. B, BIOINTERFACES, Bd. 107, 1. Juli 2013 (2013-07-01), Seiten 160-166, XP55283077, NL ISSN: 0927-7765, DOI: 10.1016/j.colsurfb.2013.02.001
- DATABASE EMBASE [Online] ELSEVIER SCIENCE PUBLISHERS, AMSTERDAM, NL; 1. Oktober 2002 (2002-10-01), SILVERBERG D ET AL: "In vitro analysis of antifungal impregnated polymethylmethacrylate bone cement", XP002759231, Database accession no. EMB-2002367074 & SILVERBERG D ET AL: "In vitro analysis of antifungal impregnated polymethylmethacrylate bone cement", CLINICAL ORTHOPAEDICS AND RELATED RESEARCH 20021001 US, Nr. 403, 1. Oktober 2002 (2002-10-01), Seiten 228-231, ISSN: 0009-921X
- Y. H. CHANG ET AL: "Liquid antibiotics in bone cement: an effective way to improve the efficiency of antibiotic release in antibiotic loaded bone cement", BONE AND JOINT RESEARCH, Bd. 3, Nr. 8, 7. August 2014 (2014-08-07), Seiten 246-251, XP055283243, DOI: 10.1302/2046-3758.38.2000305
- BRIAN CUNNINGHAM ET AL: "Liposomal Formulation Increases Local Delivery of Amphotericin from Bone Cement: A Pilot Study", CLINICAL ORTHOPAEDICS AND RELATED RESEARCHÂ TM, SPRINGER-VERLAG, NEW YORK, Bd. 470, Nr. 10, 31. März 2012 (2012-03-31), Seiten 2671-2676, XP035111904, ISSN: 1528-1132, DOI: 10.1007/S11999-012-2317-4

## Beschreibung

Es wird ein antimykotisch wirksamer Knochenzement basierend auf organischen Polymeren, wie Polymethylmethacrylat, beschrieben. Der Knochenzement umfasst ein Antimykotikum, insbesondere Amphotericin B, das in Gegenwart von Wasser oder wässrigen Medien, wie Köperflüssigkeit aus dem polymerisierten Knochenzement freigesetzt wird. Das Antimykotikum liegt partikulär vor und ist mit einem Gemisch aus mindestens einem 1-Methylamino-1-desoxy-zuckeralkohol und mindestens einer Fettsäure umhüllt. Gleichfalls wird ein Verfahren zur Herstellung des Gemisches umfassend ein Antimykotikum, wie Amphotericin B-Partikel, und 1-Methylamino-1-desoxy-zuckeralkohol und mindestens eine Fettsäure vorgeschlagen. Das Antimykotikum ist umhüllt von 1-Methylamino-1-desoxy-zuckeralkohol und mindestens einer Fettsäure. Gegenstand der Erfindung ist ein antimykotischer Knochenzement, der für die mechanische Verankerung von Revisions-Gelenkendoprothesen im Rahmen von septischen Revisionen vorgesehen ist, bei denen pathogene Mikroorganismen aus der Gruppe der Fungi, insbesondere der Hefen und der Aspergillen, ursächlich sind. Daneben ist der antibiotische Polymethylmethacrylat-Knochenzement auch für die Herstellung von Spacern als temporäre Platzhalter bei zweiseitigen septischen Revisionen geeignet.

Gelenkendoprothesen werden in grossem Umfang bei unterschiedlichsten Gelenkerkrankungen mit sehr grossem Erfolg zur Erhaltung der Bewegungsfunktionen der Patienten eingesetzt. Bei Patienten mit geschwächtem Immunsystem, beispielsweise bei Infektionen mit HIV, nach Organtransplantationen und bei Krebserkrankungen, kann es zu Infektionen von Gelenkendoprothesen durch Mikroorganismen aus der Gruppe der Fungi kommen. Diese Erkrankungen sind extrem selten und auch sehr schwierig zu therapieren.

Bei septischen Revisionen, bei denen bakterielle Erreger ursächlich sind, werden zur Behandlung dieser Infektionen der einseitige und auch der zweiseitige Gelenkendoprothesenwechsel durchgeführt. Für die dauerhafte mechanische Fixierung der Revisions-Gelenkendoprothesen haben sich Revisions-Polymethylmethacrylat-Knochenzemente bewährt, die ein Antibiotikum oder zwei oder auch mehrere Antibiotika enthalten. Durch diese Antibiotika wird die Revisions-Gelenkendoprothese und das umgebende Knochen- und Weichgewebe zumindest unmittelbar postoperativ vor einer erneuten mikrobiellen Besiedlung geschützt. Neben der individuellen Beimischung von Antibiotika durch den Arzt haben sich industriell hergestellte Revisions-Polymethylmethacrylat-Knochenzemente bewährt.

In den seltenen Fällen, bei denen Fungi ursächlich für eine Infektion von Gelenkendoprothesen sind, steht bisher kein industriell gefertigter, homogener Revisions-Polymethylmethacrylat-Knochenzement zur Verfügung, bei dem wirksame Mengen eines Antimykotikums bei Einwirkung von Körperflüssigkeiten, wie Wundsekret und Blut, freigesetzt werden. Deshalb wurden bisher in der Klinik übliche Antimykotika normalem Polymethylmethacrylat-Knochenzementpulver beigemischt. Problematisch ist dabei, dass die Mehrzahl der üblichen Antimykotika, beispielweise der Triazol-Antimykotika, in Wasser nur sehr gering löslich ist und im humanen Organismus eine hohe Proteinbindung zeigt. Aus diesem Grund ist die Herauslösung üblicher Antimykotika aus hydrophobem Polymethylmethacrylat-Knochenzement sehr problematisch und die Freisetzung antimykotisch wirksamer Wirkstoffmengen in Frage gestellt.

Das Antimykotikum Amphotericin B (CAS 1397-89-3) ist ein Polyen-Makrolacton, das aus dem Streptomyceten *Streptomyces nodosum* isoliert wurde. Es bindet an das für Fungi typische Steroid Ergosterin, einem Bestandteil der Zellmembran von Fungi. Die fungizide Wirkung von Amphotericin B wird auf eine Permeabilitätserhöhung der Zellmembranen für Kalium-Ionen zurückgeführt (M. Baginski, J. Czub: Amphotericin B and Its New Derivatives-Mode of Action. Current Drug Metabolism 10 (5) (2009) 459-69.). Besonders vorteilhaft ist die sehr breite antimykotische Wirksamkeit von Amphotericin B gegen eine Vielzahl von human-pathogenen Fungi, einschliesslich von Candida-Spezies. Amphotericin B ist in Wasser im pH-Bereich von 6 bis 7 nur gering löslich. Für die systemische Anwendung von Amphotericin B im menschlichen Organismus wurde daher versucht, eine in Wasser zu mindestens teilweise lösliche Zubereitung herzustellen. Dazu wurden Amphotericin B-Emulsionen mit Lipiden entwickelt (EP0317120). Weiterhin sind Addukte mit Dinatriumdesoxycholat (Barner et al., Antibiotics Annual 1957-1958: 53-58.) und mit Natriumcholesterylsulfat (EP0303683) bekannt. Diese Emulsionen und auch Addukte werden mit in Wasser oder organischen Lösungsmitteln gelöstem Amphotericin B hergestellt. Diese Lösungsmittel-abhängigen Prozesse erfordern einen hohen materiellen und zeitlichen Aufwand.

DE133016A1 offenbart ein Verfahren zur Herstellung einer Tricalziumphosphat-Knochenkeramik zur Verwendung als Knochenimplantat, wobei nach dem Brennvorgang die Keramik mit einer Wirkstoff enthaltenden Lösung behandelt wird und anschliessend getrocknet wird. EP 0642363B1 offenbart eine mit einem Hydrogel überzogene orthopädische Befestigungsvorrichtung, wobei das Hydrogel mit einem pharmazeutischen Wirkstoff imprägniert ist. Das Hydrogel dehnt sich bei Anwesenheit von Wasser aus. Nachteilig an den beiden letztgenannten Anwendungen ist, dass der Wirkstoff nur auf vorgefertigten keramischen Implantaten und auch nur oberflächlich auf das Implantat imprägniert werden kann.

C. Salerno et al. (Colloids and Surfaces B: Biointerphases 107 (2013), 160-166) beschreibt Formulierungen von Amphotericin B mit 12-Hydroxystearinsäure und Ethanolamin.

B. Cunningham et al. (Clin. Orthop. Relat. Res. (2012) 470, 2671-2676) beschreibt Knochenzemente mit liposomalen Amphotericin-Formulierungen. Eine Aufgabe der Erfindung bestand darin, einen antimykotisch wirksamen Knochenzement basierend auf organischen Polymeren und Monomeren, wie Polymethylmethacrylat-Knochenzement, zu entwickeln. Des Weiteren bestand die Aufgabe, dass aus dem polymerisierten Knochenzement das Antimykotikum, vorzugsweise Amphotericin B, in einer antimykotisch wirksamen Menge bei Einwirkung von wässrigen Flüssigkeiten freigesetzt wird. Ferner wurde sich die Aufgabe gestellt, die Wasserlöslichkeit des Antimykotikums ohne Veränderungen seiner Struktur für die vorgenannten Zwecke anzupassen. Ferner sollen die zur Erhöhung der Wasserlöslichkeit verwendeten Additive kostengünstig sein. Zudem soll ein einfaches und kostengünstiges Verfahren entwickelt werden, in dem das Antimykotikum, vorzugsweise Amphotericin B, mit mindestens einem Additiv ohne Verwendung weiterer Kontaminationen, wie Lösemitteln, zur Verbesserung seiner Löslichkeit in Wasser modifiziert werden kann.

Gelöst werden die Aufgaben der Erfindung gemäss Ansprüche 1, 10, 11, 13-15 sowie durch ein Verfahren nach Anspruch 9, als auch durch das Kit nach Anspruch 12 und dessen Verwendung.

Vorteilhafte Ausführungsformen sind in den Unteransprüchen dargestellt sowie detailliert in der Beschreibung erläutert.

Gegenstand der Erfindung ist ein antimykotischer Polymethylmethacrylat-Knochenzement der Methylmethacrylat, mindestens ein Polymethylmethacrylat oder ein Polymethylmethacrylat-Copolymer, mindestens einen radikalischen Initiator, mindestens einen Akzellerator und mindestens einen Röntgenopaker umfasst, wobei die Komponenten in einer Alternative in einer pulverförmigen Komponente und einer flüssigen Monomerkomponente oder in zwei bei Raumtemperatur pastenförmigen Komponenten vorliegen können. Der Knochenzement umfasst erfindungsgemäss Partikel enthaltend Amphotericin B, wobei das Amphotericin B mit einem Gemisch aus mindestens einem 1-Methylamino-1-desoxy-zuckeralkohol und mindestens einer Fettsäure umhüllt ist.

Überraschend wurde gefunden, dass ein Polymethylmethacrylat-Knochenzement der Amphotericin B, insbesondere Amphotericin B Partikel, enthält, die mit einem Gemisch aus einem Methylamino-1-desoxy-zuckeralkohol und einer Fettsäure umhüllt sind, in Gegenwart von wässrigen Lösungen antimykotisch wirksame Mengen an Amphotericin B abgibt.

Gegenstand der Erfindung ist ein polymerisierbarer Knochenzement umfassend
(i) mindestens ein radikalisch polymerisierbares Monomer, insbesondere ein flüssiges Monomer,
(ii) mindestens ein in (i) lösliches organisches Polymer, insbesondere mindestens ein Polymethacrylat oder ein Polymethacrylat-Copolymer, und
(ii) mindestens einen Polymerisaitonsinitiator,
(iv) mindestens einen Röntgenopaker, optional
(v) mindestens ein Polymerisationsbeschleuniger, wobei der Knochenzement mindestens ein Antimykotikum, vorzugsweise Amphotericin B, im Gemisch mit mindestens einem Amino-funktionellen Lösungsvermittler und mindestens einer Carbonsäure als Lösungsvermittler umfasst, vorzugsweise liegt das Antimykotikum im Gemisch mit zwei Lösungsvermittlern als Partikel, insbesondere partikulär vor, wobei das Antimykotikum umhüllt ist von 1-Methylamino-1-desoxy-zuckeralkohol und mindestens einer Fettsäure.
Vorteil des erfindungsgemässen Knochenzementes ist, dass das Antimykotikum sowohl in der Matrix des polymerisierbaren Knochenzementes als auch in der Matrix des ausgehärteten Knochenzementes verteilt vorliegt, vorzugsweise annähernd homogen verteilt. Ein wesentlicher Vorteil des erfindungsgemässen Knochenzementes liegt darin, dass er als ein verarbeitungsfähiger Knochenzement bereitgestellt wird, der nach der Applikation polymerisiert wird oder auch zu chirurgischen Implantaten oder Teilen davon verarbeitet werden kann.

Entsprechend einer besonders bevorzugten Ausführungsform kann der Knochenzement als 2K-System vorliegen. In den 2K-Systemen kann der Knochenzement als Paste/Paste, als Pulver/Paste bzw. Pulver/Flüssigkeit Komponenten vorliegen. Zur Herstellung des polymerisierbaren Knochenzementes werden die beiden Komponenten miteinander gemischt und können verarbeitet werden. Mit dem erfindungsgemässen Knochenzement können auf einfache und wirtschaftliche Weise jegliche Anwendungsfelder des Knochenzementes auch für antimykotische Anwendungen eröffnet werden.

Daher ist nach einer besonders bevorzugten Ausführungsform Gegenstand der Erfindung ein Knochenzement umfassend zwei insbesondere voneinander getrennt vorliegende Komponenten, vorzugsweise Komponenten A und B, wobei
(i) Komponente A als Paste vorliegt und umfasst (a1) mindestens ein radikalisch polymerisierbares Monomer, (a2) mindestens ein in (a1) lösliches organisches Polymer, und (a3) mindestens einen Polymerisationsinitiator; und
   Komponente B als Paste vorliegt und umfasst (b1) mindestens ein radikalisch polymerisierbares Monomer, (b2) mindestens ein in (i), (b1) lösliches organisches Polymer, und (b3) mindestens einen Polymerisationsbeschleuniger; oder
(ii) Komponente A als Pulver vorliegt und umfasst (a1) mindestens ein pulverförmiges Polyacrylat, (a2) mindestens einen pulverförmigen Röntgenopaker, und (a3) mindestens einen Polymerisationsinitiator; und
   Komponente B als Flüssigkeit oder Paste vorliegt und umfasst (b1) mindestens ein radikalisch polymerisierbares Monomer, (b2) optional mindestens ein in (ii), (a1) lösliches organisches Polymer, und (b3) mindestens einen Polymerisationsbeschleuniger, und wobei mindestens die Komponente A und/oder die Komponente B als (a4) und/oder (b4) mindestens ein Antimykotikum, insbesondere ein Antimykotikum mit mindestens einem, vorzugsweise zwei, Lösungsvermittler umfasst. Bevorzugt liegt das Antimykotikum als (a4) und/oder (b4) im Gemisch mit zwei Lösungsvermittlern vor. Dabei ist es besonders bevorzugt, wenn das Antimykotikum und die Lösungsvermittler als ein partikuläres Gemisch vorliegen. Ohne an die Theorie gebunden zu sein wird angenommen, dass die Lösungsvermittler das Antimykotikum umhüllen. Lösungsvermittler sind 1-Methylamino-1-desoxy-zuckeralkohol sowie Carbonsäuren. Vorzugsweise werden hydrophile Amino-funktionelle Lösungsvermittler und lipophile Carbonsäuren als Lösungsvermittler zusammen in einem Gemisch von Lösungsvermittlern verwendet.

Bevorzugt bilden die mindestens zwei Lösungsvermittler ein Säure-Base-Addukt das in Gegenwart wässeriger Lösungen, insbesondere Körperflüssigkeiten, in diesen löslich ist. Des Weiteren wird angenommen, ohne an diese Theorie gebunden zu sein, dass die Lösungsvermittler in der Lage sind das Antimykotikum, insbesondere Amphotericin B, zu umhüllen oder einzubetten, vorzugsweise erfolgt eine Art Maskierung und/oder Komplexierung des schwer wasserlöslichen Antimykotikums.

Der Wirkstoff Amphotericin B mit der CAS-Nr. 1397-89-3 wird auch als 3-(4-Amino-3,5-dihydroxy-6-methyl-oxan-2-yl)oxy-19,25,27,30,31,33,35,37-octahydroxy-18,20,21-trimethyl-23-oxo-22,39-dioxabicyclo[33.3.1]nonatriaconta-4,6,8,10,12,14,16-heptaen-38-carbonsäure bezeichnet.

Die Lösungsvermittler dienen dazu ein schwer in wässrigen Medien lösliches Antimykotikum in die wässrige Phase zu überführen. Es hat sich herausgestellt, dass ein Gemisch von mindestens zwei Lösungsvermittlern, umfassend einen hydrophilen Amino-funktionellen Lösungsvermittler und eine hydrophile Alkyl- und/oder Aryl-funktionelle Carbonsäure als zweiter Lösungsvermittler, das Antimykotikum besonders gut aus den polymerisierten Knochenzementen in Gegenwart von wässrigen Medien freisetzen kann. Des Weiteren kann dieses Gemisch umfassend Antimykotikum und Lösungsvermittler im ungehärteten Knochenzement homogen dispergiert werden. Ein weiterer Vorteil ist die lösemittelfreie Herstellbarkeit des Gemisches. Das Antimykotikum liegt im Gemisch mit mindestens einem Amino-funktionellen Lösungsvermittler vor, wobei der Lösungsvermittler 1-Methylamino-1-desoxy-zuckeralkohol, wie N-Methyl-Glucamin ist. Gleichfalls bevorzugt ist 1-Desoxy-1-methylaminosorbit (N-Methyl-D-Glucamin). Erfindungsgemäss wird N-Methyl-D-Glucamin (1-Desoxy-1-methylaminosorbit, CAS 6284-40-8) als 1-Methylamino-1-desoxy-zuckeralkohol bevorzugt. Für die Herstellung von 1-Methylamino-1-desoxy-zuckeralkoholen kommen sämtliche Aldosen in Frage, die mit Methylamin am Kohlenstoffatom 1 umgesetzt und anschliessend hydriert werden können. Dabei werden Allose, Altrose, Mannose, Gulose, Idose, Galactose, Talose, Ribose, Arabinose, Xylose, Lyxose, Erythrose und Threose als Aldosen bevorzugt. Daneben ist es auch prinzipiell möglich die hydrierten Umsetzungsprodukte von Ketosen mit Methylamin einzusetzen. Das Antimykotikum liegt im Gemisch mit mindestens einer Carbonsäure als Lösungsvermittler vor, insbesondere einer lipophilen Carbonsäure. Vorzugsweise umfasst der Lösungsvermittler Carbonsäuren mit Alkyl- und/oder Aryl-Resten, wobei die Carbonsäure mindestens 8 C-Atome aufweist und vorzugsweise eine gerade Anzahl an C-Atomen hat. Besonders bevorzugt sind 8 bis 50 C-Atome, vorzugsweise 8 bis 24 C-Atome, weiter bevorzugt sind Alkan-mono-Carbonsäuren, besonders bevorzugt sind gesättigte Fettsäuren. Als besonders bevorzugte Carbonsäure seien genannt Palmitinsäure, Laurinsäure, Myristinsäure, Stearinsäure, Nonansäure, Decansäure, Undecansäure, Tridecansäure, Pentadecansäure, Heptadecansäure, Octadecansäure, Nonadecansäure, Eicosan-/Icosansäure, Heneicosansäure, Docosansäure, Tetracosansäure, Hexacosansäure, ohne dass die Carbonsäuren auf diese beschränkt sein sollen. Gleichfalls können Aryl-funktionelle Carbonsäuren wie Benzoesäure, Phenylessigsäure oder andere pharmakologischen Carbonsäuren als Lösungsvermittler eingesetzt werden.

Aufgrund der biologischen Abbaubarkeit werden gesättigte Fettsäuren mit einer geraden Anzahl von Kohlenstoff-Atomen bevorzugt, wobei Stearinsäure, Palmitinsäure, Myristinsäure und Laurinsäure besonders bevorzugt sind. Daneben ist es auch möglich, andere schmelzbare oder auch bei Raumtemperatur flüssige Fettsäuren zu verwenden, sofern die Fettsäuren mit dem Amino-funktionellen Lösungsvermittler einen Feststoff bilden. Das Antimykotikum, vorzugsweise Amphotericin B, ist umhüllt von einem 1-Methylamino-1-desoxy-zuckeralkohol und mindestens einer Fettsäure, besonders bevorzugt ist Amphotericin B, umhüllt von N-Methyl-D-Glucamin und mindestens einer Fettsäure.

Als besonders bevorzugte Gewichtsverhältnisse von Antimykotikum, insbesondere Amphotericin B, zur Fettsäure und zum 1-Methylamino-1-desoxy-zuckeralkohol hat sind ein Gewichtsverhältnis von um 1,0 zu 1,0 zu 1,5 bis 1,0 zu 6,0 zu 6,0 erwiesen, wobei bevorzugt das Gewichtsverhältnis von Amphotericin B zur Fettsäure und zum 1-Methylamino-1-desoxy-zuckeralkohol um 1,0 zu 1,7 zu 1,7 ist.

Ferner kann es bevorzugt sein, wenn das Antimykotikum Amphotericin B ist und im Gemisch von Lösungsvermittlern umfassend mindestens einen 1-Methylamino-1-desoxy-zuckeralkohol und mindestens eine Carbonsäure vorliegt, wobei das Gewichtsverhältnis von Amino-funktionellem Lösungsvermittler und Carbonsäuren von 1,5 zu 1,0 bis 1,0 zu 1,0 beträgt. Alternativ beträgt das Stoffmengenverhält von Amino-funktionellem Lösungsvermittler und Carbonsäuren von um 1,5 zu 1,0 bis 1,0 zu 1,0, besonders bevorzugt 1,0 zu 1,7 zu 1,7.

Weiter ist es bevorzugt, wenn das Gewichtsverhältnis von Antimykotikum, insbesondere von Amphotericin B, zu Amino-funktionellem Lösungsvermittler und Carbonsäuren von um 1,0 zu 1,5 zu 1,0 bis 1,0 zu 6,0 zu 6,0 beträgt, insbesondere etwa 1,0 zu 1,7 zu 1,7. Nach einer Alternative beträgt das Stoffmengenverhältnis des Antimykotikums, insbesondere von Amphotericin B, zu Amino-funktionellem Lösungsvermittler und Carbonsäuren von um 1,0 zu 1,5 zu 1,0 bis 1,0 zu 6,0 zu 6,0, besonders bevorzugt ist ein Verhältnis von etwa 1,0 zu 1,7 zu 1,7. Dabei kann sowohl das Gewichtsverhältnis als auch das Stoffmengenverhältnis jeweils von um +/- 0,25 bis +/- 0,5 abweichen.

Gleichfalls Gegenstand der Erfindung ist ein Verfahren zur Herstellung eines Zwischenproduktes zur Herstellung des polymerisierbaren Knochenzementes sowie das Zwischenprodukt erhältlich nach dem Verfahren, indem mindestens zwei Lösungsvermittler umfassend einen Amino-funktionellen Lösungsvermittler und mindestens eine Carbonsäure oder ein Gemisch an Lösungsvermittlern umfassend einen Amino-funktionellen Lösungsvermittler und mindestens eine Carbonsäure und ein Antimykotikum gemischt werden, wobei es sich bei dem Amino-funktionellen Lösungsvermittler um 1-Methylamino-1-desoxy-zuckeralkohol handelt. Dabei ist es besonders bevorzugt, wenn das Mischen ohne Gegenwart eines Lösemittels oder ohne weitere Zusätze erfolgt. Bevorzugt ist nach dem Verfahren ein partikuläres Zwischenprodukt erhältlich. Die Partikelgrösse des Zwischenproduktes liegt vorzugsweise im Bereich von gleich 250 µm, insbesondere von 100 nm bis 250 µm, bevorzugt von 50 bis 250 11m.

Gegenstand der Erfindung ist somit auch ein Zwischenprodukt, insbesondere ein partikuläres Zwischenprodukt, umfassend mindestens einen Amino-funktionellen Lösungsvermittler und mindestens eine Carbonsäure mit Alkyl- und/oder Aryl-Resten, wobei die Carbonsäure mindestens 8 C-Atome aufweist und umfassend ein Antimykotikum, wobei es sich bei dem Amino-funktionellen Lösungsvermittler um 1-Methylamino-1-desoxy-zuckeralkohol handelt. Das Verfahren wird durchgeführt, indem (i) mindestens ein oder zwei Lösungsvermittler oder ein Gemisch an Lösungsvermittlern geschmolzen werden und zu dem geschmolzenen Gemisch ein Antimykotikum zugefügt wird. Das erfindungsgemässe Verfahren ist ein besonders umweltfreundliches Verfahren, da es gänzlich auf den Einsatz von Lösemitteln verzichtet. Zudem ist das erfindungsgemässe Verfahren besonders wirtschaftlich. Die erhaltene Mischung wird abgekühlt und zerkleinert. Die Zerkleinerung kann mechanisch beispielsweise durch Mahlen, Schredder, Schneiden etc. erfolgen. Ebenso kann die warne Schmelze oder die abgekühlte Schmelze extrudiert werden. Nachfolgend wird die Mischung in eine übliche Zusammensetzung eines polymerisierbaren Knochenzementes eingearbeitet.

Der so hergestellte, insbesondere der nach dem Verfahren erhältliche, polymerisierbare Knochenzement, ist aufgrund der spezifisch gewählten Lösungsvermittler physiologisch verträglich und ermöglicht die spätere Freisetzung, insbesondere eine retardierte Freisetzung des Antimykotikums, aus dem dann polymerisierten Knochenzement.

Der in dem Verfahren eingesetzte Lösungsvermittler umfasst mindestens einen Amino-funktionellen Lösungsvermittler und mindestens eine Carbonsäure mit Alkyl-und/oder Aryl-Resten, wobei die Carbonsäure mindestens 8 C-Atome aufweist und wobei es sich bei dem Amino-funktionellen Lösungsvermittler um 1-Methylamino-1-desoxyzuckeralkohol handelt. Die Herstellung erfolgt indem (i) a) mindestens ein Amino-funktioneller Lösungsvermittler und/oder mindestens eine Carbonsäure mit Alkyl- und/oder Aryl-Resten, wobei die Carbonsäure mindestens 8 C-Atome aufweist, geschmolzen werden und zu dem geschmolzenen Gemisch als Antimykotikum Amphotericin B zugefügt wird, wobei es sich bei dem Amino-funktionellen Lösungsvermittler um 1-Methylamino-1-desoxy-zuckeralkohol handelt. In nachfolgenden Schritten wird die erhaltene Schmelze abgekühlt und die erstarrte Schmelze zerkleinert oder alternativ wird die formbare Schmelze extrudiert. Alternativ kann b) das geschmolzene Gemisch umfassend Amphotericin B abgekühlt werden und optional durch Einwirken mechanischer Energie zerkleinert, bspw. gemahlen werden.

Gegenstand der Erfindung ist daher auch ein Verfahren zur Herstellung von Partikeln umfassend Amphotericin B und -1-Methylamino-1-desoxy-zuckeralkohol und eine Fettsäure. Das erfindungsgemässe Verfahren kann auch wie folgt durchgeführt werden: Der 1-Methylamino-1-desoxy-zuckeralkohol wird bei einer Temperatur grösser 100 °C geschmolzen. Der Schmelze wird bei einer Temperatur grösser 100 °C mindestens eine Fettsäure zugesetzt und optional gemischt. Zu der erhaltenen Schmelze wird das Antimykotikum zugesetzt, insbesondere wird Amphotericin B zugesetzt, und optional gemischt. Die erhaltene Schmelze wird abgekühlt, vorzugsweise auf Raumtemperatur.

Die erkaltete Schmelze ist fest und wird bei Raumtemperatur zu Partikeln mit einer Siebfraktion von kleiner gleich 250 µm gemahlen.

Im Schritt der Zugabe und des Vermischens des Antimykotikums ist es wichtig, dass die Schmelze möglichst umgehend nach der Vermischung mit dem Antimykotikum, insbesondere mit Amphotericin B, auf Raumtemperatur abgekühlt wird, so dass die thermische Belastung des Amphotericin B nur wenige Sekunden bis Minuten andauert. Der besondere Vorteil des erfindungsgemässen Verfahrens besteht darin, dass innerhalb kurzer Zeit mit geringstem Aufwand, ohne Verwendung von Lösungsmittel, umhüllte Amphotericin B-Partikel hergestellt werden können. Die Abkühlung kann auch durch Einleiten oder Extrudieren der Schmelze in flüssigen Stickstoff erfolgen.

Gegenstand der Erfindung ist ebenfalls eine Zusammensetzung zur Verwendung zur Behandlung und/oder Prävention von Mykosen, wobei die Zusammensetzung umfasst
(i) mindestens ein radikalisch polymerisierbares Monomer, insbesondere flüssiges Monomer,
(ii)mindestens ein in (i) lösliches organisches Polymer, insbesondere mindestens ein Polymethacrylat, oder ein Polymethacrylat-Copolymer, und
(iii) mindestens einen Polymerisationsinitiator, und mindestens ein Antimykotikum, und optional
(iv) mindestens einen Röntgenopaker,
(v) mindestens einen Polymerisationsbeschleuniger, wobei das Antimykotikum im Gemisch mit mindestens zwei Lösungsvermittlern, umfassend einen Amino-funktionellen Lösungsvermittler und mindestens eine Carbonsäure vorliegt, wobei das Antimykotikum umhüllt ist von 1-Methylamino-1-desoxy-zuckeralkohol.

Gleichfalls Gegenstand der Erfindung ist ein Kit zur Herstellung von polymerisierbarem Knochenzement, umfassend die voneinander getrennt vorliegenden Komponenten A und B. Somit ist Gegenstand der Erfindung ein Kit umfassend die Komponenten A und B, wobei
(i) Komponente A als Paste vorliegt und umfasst (a1) mindestens ein radikalisch polymerisierbares Monomer, (a2) mindestens ein in (a1) lösliches organisches Polymer, und (a3) mindestens einen Polymerisationsinitiator beinhaltet; und
   Komponente B als Paste vorliegt und umfasst (b1) mindestens ein radikalisch polymerisierbares Monomer, (b2) mindestens ein in (i), (b1) lösliches organisches Polymer, und (b3) mindestens einen Polymerisationsbeschleuniger beinhaltet; oder
(ii) Komponente A als Pulver vorliegt und umfasst (a1) mindestens ein pulverförmiges Polyacrylat, (a2) mindestens einen pulverförmigen Röntgenopaker, und (a3) mindestens einen Polymerisationsinitiator beinhaltet; und
   Komponente B als Flüssigkeit oder Paste vorliegt und umfasst (b1) mindestens ein radikalisch polymerisierbares Monomer, (b2) optional mindestens ein in (ii), (a1) lösliches organisches Polymer, und (b3) mindestens einen Polymerisationsbeschleuniger beinhaltet; und, wobei mindestens die Komponente A und/oder B als (a4) und/oder (b4) mindestens ein Antimykotikum mit mindestens zwei Lösungsvermittler umfassend einen Amino-funktionellen Lösungsvermittler und mindestens eine Carbonsäure, wobei das Antimykotikum umhüllt ist von 1-Methylamino-1-desoxy-zuckeralkohol und mindestens einer Fettsäure.
Gemäss einer bevorzugten Ausführungsform wird ein Knochenzement oder Kit beansprucht, der die folgenden Komponenten umfasst, wobei
(i) Komponente A als Paste vorliegt und umfasst
   (a1) mindestens ein radikalisch polymerisierbares Monomer, insbesondere zu 15 bis 85 Gew.-%, vorzugsweise 20 bis 70 Gew.-%, bevorzugt 25 bis 60 Gew.-%, besonders bevorzugt 25 bis 50 Gew.-%,
   (a2) mindestens ein in (a1) lösliches organisches Polymer, insbesondere zu 5 bis 50 Gew.-%, bevorzugt 10 bis 40 Gew.-%, besonders bevorzugt 20 bis 30 Gew.-%, und
   (a3) mindestens einen Polymerisationsinitiator beinhaltet, insbesondere zu 0,01 bis 10 Gew.-%, bevorzugt 0,01 bis 8 Gew.-%, besonders bevorzugt 0,01 bis 5 Gew.-%; und
   Komponente B als Paste vorliegt und umfasst
   (b1) mindestens ein radikalisch polymerisierbares Monomer, insbesondere zu 15 bis 85 Gew.-%, vorzugsweise 20 bis 70 Gew.-%, bevorzugt 25 bis 60 Gew.-%, besonders bevorzugt 25 bis 50 Gew.-%,
   (b2) mindestens ein in (i), (b1) lösliches organisches Polymer, insbesondere zu 5 bis 50 Gew.-%, bevorzugt 10 bis 40 Gew.-%, besonders bevorzugt 20 bis 30 Gew.-%, und
   (b3) mindestens einen Polymerisationsbeschleuniger; insbesondere zu 0,0005 bis 0,5 Gew.-%; oder
(ii) Komponente A als Pulver vorliegt und umfasst
   (a1) mindestens ein pulverförmiges Polyacrylat, insbesondere zu 1 bis 95 Gew.-%, bevorzugt 15 bis 85 Gew.-%,
   (a2) mindestens einen pulverförmigen Röntgenopaker, insbesondere zu 3 bis 60 Gew.-%, bevorzugt 3 bis 30 Gew.-%, und
   (a3) mindestens einen Polymerisationsinitiator; insbesondere zu 0,01 bis 10 Gew.-%, bevorzugt 0,01 bis 8 Gew.-%, besonders bevorzugt 0,01 bis 5 Gew.-%, und
   Komponente B als Flüssigkeit oder Paste vorliegt und umfasst
   (b1) mindestens ein radikalisch polymerisierbares Monomer, insbesondere zu 15 bis 85 Gew.-%, vorzugsweise 20 bis 70 Gew.-%, bevorzugt 25 bis 60 Gew.-%, besonders bevorzugt 25 bis 50 Gew.-%,
   (b2) optional mindestens ein in (ii), (a1) lösliches organisches Polymer, insbesondere zu 5 bis 50 Gew.-%, bevorzugt 10 bis 40 Gew.-%, besonders bevorzugt 20 bis 30 Gew.-%, und
   (b3) mindestens einen Polymerisationsbeschleuniger; insbesondere zu 0,0005 bis 0,5 Gew.-%, und,
   wobei mindestens die Komponente A und/oder die Komponente B als (a4) und/oder (b4) mindestens ein Antimykotikum und mindestens zwei Lösungsvermittler, umfassend einen Amino-funktionellen Lösungsvermittler und mindestens eine Carbonsäure umfasst, wobei das Antimykotikum umhüllt ist von 1-Methylamino-1-desoxy-zuckeralkohol und mindestens einer Fettsäure.
Die Komponenten (a4) und/oder (b4) sind in der jeweiligen Komponente, wie der Pulverkomponente A oder der Paste A und/oder B vorzugsweise von 0,01 bis 2,0 Gew.-%, insbesondere von 0,01 Gew.-% bis 1,0 Gew.-%, vorzugsweise von 0,05 bis 0,5 Gew.-% enthalten. Dabei ist es bevorzugt, wenn das Antikykotikum in beiden Pasten enthalten ist. In der Gesamtzusammensetzung des Knochenzementes liegt der Gehalt an Antimykotikum vorzugsweise im Bereich von 0,001 bis 2,0 Gew.-%, bevorzugt von 0,001 bis 1,0 Gew.-%, besonders bevorzugt im Bereich von 0,05 bis 0,5 Gew.-%.

Wobei die Pulverkomponente A und die Flüssigkeit A oder Paste A vorzugsweise im Verhältnis von annähernd 2 zu 1 bis 1 zu 2 gemischt werden. Grundsätzlich könnten die Pasten A und B in jedem beliebigen Verhältnis miteinander gemischt werden, wobei sich die Verwendung der Pasten A und B in einem Verhältnis von im Wesentlichen 1 zu 1 zur Herstellung einer Mischung als bevorzugt erwiesen hat, wobei das Verhältnis zueinander unabhängig plus/minus 50 % schwanken kann.

Ferner ist Gegenstand der Erfindung ein polymeriserbarer bzw. härtbarer Knochenzement erhältlich durch Vermischen der Komponenten A und B. Ebenso ist Gegenstand der Erfindung ein polymerisierter bzw. gehärteter Knochenzement zur Behandlung und/oder Prävention von Mykosen, wobei das Antimykotikum in Gegenwart von Feuchte, Wasser, wässrigem Milieu, wie Körperflüssigkeiten, oder einer wässrigen Lösung, freigesetzt wird, insbesondere wird das Antimykotikum retardiert freigesetzt. Darüber hinaus ist Gegenstand der Erfindung ein Formkörper erhältlich durch Formen und Polymerisation des härtbaren (synonym zu polymerisierbaren) Knochenzements.

Nach einer weiteren Ausführungsform ist Gegenstand der Erfindung ein chirurgisches Implantat zur Verwendung zur Behandlung und/oder Prävention einer Mykose, insbesondere als chirurgisches Implantat oder Teil eines Implantates, antimykotisches Implantat, Revisionsimplantat, Schraube, Nagel, chirurgische Platte, zur mechanischen Fixierung primärer Totalgelenkendoprothesen, zur mechanischen Fixierung von Revisions-Totalgelenkendoprothesen, zur Augmentation von osteoporotischem Knochengewebe und ganz besonders bevorzugt für die Vertebroplastie, Kyphoplastie und die Augmentation von Bohrlöchern in osteoporotischem Knochengewebe, zur Auffüllung von Knochenkavitäten, zur Femuroplastie, zur Herstellung von Spacern, zur mechanischen Fixierung von Gelenkprothesen, zur Abdeckung von Schädeldefekten oder zur Herstellung von Trägermaterialien für die lokale Antibiotika-Therapie oder als Trägermaterial für eine lokale Freisetzung von pharmazeutisch wirksamen Substanzen.

Nach einer Ausführungsform ist Gegenstand der Erfindung ein Knochenzement umfassend als Monomer mindestens einen Alkyl-2-acrylsäurealkylester, Aryl-2-acrylsäurealkylester, Arylalkyl-2-acrylsäurealkylester, jeweils unabhängig mit 1 bis 20 C-Atomen in der Alkyl-Gruppe, jeweils unabhängig mit 6 bis 14 C-Atomen in der Aryl-Gruppe, jeweils unabhängig mit 6 bis 14 C-Atomen in der Arylalkyl-Gruppe und jeweils unabhängig mit 1 bis 10 C-Atomen in der Alkylester-Gruppe oder einer Mischung umfassend mindestens zwei der genannten Monomere.

Vorzugsweise beinhaltet die erfindungsgemässe Paste das radikalisch polymerisierbare Monomer in einer Menge in einem Bereich von 15 bis 85 Gew.-%, mehr bevorzugt 20 bis 70 Gew.-%, noch mehr bevorzugt 25 bis 60 Gew.-% und besonders bevorzugt 25 bis 50 Gew.-%, jeweils bezogen auf das Gesamtgewicht der erfindungsgemässen Paste.

Das organische Polymer wird vorzugsweise ausgewählt aus mindestens einem Poly(alkyl-2-acrylsäure-alkylester), Poly(aryl-2-acrylsäurealkylester), Poly(arylalkyl-2-acrylsäurealkylester), jeweils unabhängig mit 1 bis 20 C-Atomen in der Alkyl-Gruppe, jeweils unabhängig mit 6 bis 14 C-Atomen in der Aryl-Gruppe, jeweils unabhängig mit 6 bis 14 C-Atomen in der Arylalkyl-Gruppe und jeweils unabhängig mit 1 bis 10 C-Atomen in der Alkylester-Gruppe oder einer Mischung umfassend mindestens zwei der genannten Polymere. Das organische Polymer ist vorzugsweise ausgewählt aus der Gruppe Poly(methacrylsäuremethylester), Poly(methacrylsäureethylester), Poly(methylmethacryl-säurepropylester), Poly(methacrylsäureisopropylester), Poly(methylmethacrylat-co-methylacrylat), Poly(styrol-co-methyl-meth-acrylat), Copolymeren der genannten Verbindungen und einer Mischung umfassend mindestens zwei dieser Polymere.

Gegenstand der Erfindung ist ebenfalls ein Knochenzement umfassend mindestens ein organisches Polymer, wie ein Poly(methacrylsäuremethylester) (PMMA) und Methacrylsäuremethylester (MMA) als Monomer.

Als in dem mindestens einen radikalisch polymerisierbaren Monomer lösliches Polymer wird ein Polymer verstanden, das sich in diesem polymerisierbaren Monomer zu wenigstens 10 g/l, vorzugsweise zu wenigstens 25 g/l, besonders bevorzugt zu wenigstens 50 g/l und ganz besonders bevorzugt zu wenigstens 100 g/l, löst. Das in dem polymerisierbaren Monomer lösliche Polymer kann ein Homopolymer oder ein Copolymer sein. Bei diesem löslichen Polymer handelt es sich vorzugsweise um ein Polymer mit einer gewichtsmittleren Molmasse (Mw) von wenigstens 150.000 g/mol, insbesondere mindestens 200.000 g/mol bis grösser gleich 5000.000 g/mol. Beispielsweise kann es sich bei dem löslichen Polymer um ein Polymer oder Copolymer eines Methacrylsäureesters handeln. Gemäss einer besonders bevorzugten Ausführungsform ist das wenigstens eine lösliche Polymer aus der Gruppe ausgewählt, die aus Polymethacrylsäuremethylester (PMMA), Polymethacrylsäureethylester (PMAE), Polymethacrylsäurepropylester (PMAP), Polymethacrylsäureisopropylester, Poly(methylmethacrylat-co-methylacrylat), Poly(styren-co-methylmeth-acrylat) und einer Mischung aus mindestens zwei dieser Polymere besteht.

Die Menge des in dem radikalisch polymerisierbaren Monomer löslichen Polymers in dem erfindungsgemässen Knochenzement liegt üblicherweise in einem Bereich von 1 bis 85 Gew.-%, bezogen auf das Gesamtgewicht des Knochenzementes. Entsprechend kann der Gehalt an organischem Polymer jeweils unabhängig in einer der nachstehenden Pasten A, B und/oder Flüssigkeit B sowie der Pulverkomponente A von 1 bis 85 Gew.-% in Bezug auf die jeweilige Gesamtzusammensetzung an Paste, Flüssigkeit, oder Pulverkomponente betragen.

Als in den Monomeren lösliche Polymere, insbesondere Polyacrylate, werden Polymere mit einem Molekulargewicht (Mw) von vorzugsweise grösser gleich 200.000 g/mol zur Herstellung von Pulverkomponenten eingesetzt, bevorzugt sind Molekulargewichte von grösser gleich 500.000 g/mol. Zur Verwendung in Pasten können auch Polymere mit einem Molekulargewicht von kleiner gleich 500.000 g/mol eingesetzt werden. Dabei bestimmt sich das geeignete Molekulargewicht einerseits danach, ob eine Paste oder eine Pulverkomponente hergestellt wird, sowie den weiteren Komponenten in der Paste als auch dadurch, dass das Polymer in dem eingesetzten Monomer löslich sein muss.

Ein erfindungsgemässer Knochenzement umfasst neben dem löslichen organischen Polymer, insbesondere Polymethmethacrylat (PPMA), und dem radikalisch polymerisierbaren Monomer, insbesondere Methacrylsäuremethylester, ein partikuläres, anorganisches Additiv, vorzugsweise in einer Konzentration von 0,01 bis 0,5 Gew.-%, insbesondere von 0,01 bis 0,25 Gew.-%, bevorzugt von 0,02 bis0,14 Gew.-% in Bezug auf die Gesamtzusammensetzung. Erfindungsgemäss umfasst der aus der Pulverkomponente und der flüssigen Monomerkomponente gemischte Knochenzementteig das partikuläre, anorganische Additiv in einer Konzentration von 0,02 bis0,14 Gew.-%. Zusätzlich zu den vorgenannten Komponenten umfasst ein erfindungsgemässer Knochenzement einen Röntgenopaker, einen Polymerisationsinitiator und/oder einen Polymerisationsbeschleuniger sowie optional zusätzlich Füllstoffe, die nicht dem Additiv entsprechend und lediglich verdickende Wirkung zeigen.

Das partikuläre anorganische Additiv ist ausgewählt aus der Gruppe aus pyrogenem Siliziumdioxid, pyrogenem Metall-Siliziummischoxid, Bentonit, Montmorillonit und einer Mischung enthaltend mindestens zwei der genannten Additive. Daneben ist es auch möglich, hydrophobiertes pyrogenes Siliziumdioxid einzusetzen. Die Herstellung des hydrophoben Siliziumdioxids kann nach dem Stand der Technik durch Behandlung von pyrogenem Siliziumdioxid mit Dialkyldichlorsilanen (z. B. Dimethyldichlorsilan) erfolgen.

Der erfindungsgemässe Knochenzement, die Pasten, Flüssigkeit und/oder Pulverkomponenten können mindestens einen Polymerisationsinitiator (der vorzugsweise in dem radikalisch polymerisierbaren Monomer löslich ist), wenigstens einen Polymerisationsbeschleuniger (der vorzugsweise in dem radikalisch polymerisierbaren Monomer löslich ist), gegebenenfalls wenigstens einen Co-Polymerisationsbeschleuniger oder wenigstens einen Polymerisationsinitiator, wenigstens einen Polymerisationsbeschleuniger und gegebenenfalls wenigstens einen Co-Polymerisationsbeschleuniger beinhalten.

Im Falle eines Einkomponenten-Systems als erfindungsgemässe Zusammensetzung handelt es sich bei dem Polymerisationsinitiator vorzugsweise um einen aktivierbaren Polymerisationsinitiator, z. B. um einen in der Zusammensetzung, die als Paste vorliegt, gelösten oder suspendierten Photoinitiator oder um ein in der Paste gelöstes oder suspendiertes Photoiniatorsystem. Es ist auch möglich, einen Initiator oder Initiatoren dort vorzusehen, wo er bzw. sie vorübergehend zu Kontakt mit der Paste kommt, etwa in einem Behälterteil, einer Dosiervorrichtung oder einer Transportkanüle. Auch kann im Falle eines Einkomponenten-Systems der Knochenzement oder die Paste neben dem aktivierbaren Polymerisationsinitiator auch einen elektrisch leitfähigen Röntgenopaker beinhalten. Hier eignen sich besonders Partikel von Kobalt, Eisen, NdFeB, SmCo, Kobalt-Chrom-Stahl, Zirkonium, Hafnium, Titan, Titan-Aluminium-Silizium-Legierungen und Titan-Niobium-Legierungen mit einer Partikelgrösse von 0,5-500 µm. In dem elektrisch leitfähigen Röntgenopaker können durch magnetische Wechselfelder mit einer Frequenz im Bereich von 500 Hz bis 50 kHz Wirbelströme induziert werden, die zu einer Erwärmung des Opakers führen. Durch Wärmeübertragung wird auch der Initiator aufgeheizt und zum thermischen Zerfall gebracht.

Als Polymerisationsinitiator kommen insbesondere Peroxide und Barbitursäurederivate in Betracht, wobei die Peroxide und Barbitursäurederivate vorzugsweise in dem polymerisierbaren Monomer zu wenigstens 1 g/l, vorzugsweise zu wenigstens 3 g/l, noch mehr bevorzugt zu wenigstens 5 g/l und ganz besonders bevorzugt zu wenigstens 10 g/l bei einer Temperatur von 25°C löslich sind.

Unter einem Peroxid werden erfindungsgemäss Verbindungen verstanden, die wenigstens eine Peroxogruppe (-O-O-) enthalten. Das Peroxid weist vorzugsweise keine freien aciden Gruppen auf. Bei dem Peroxid kann es sich um ein anorganisches Peroxid oder um ein organisches Peroxid handeln, wie zum Beispiel ein toxikologisch unbedenkliches Hydroperoxid. Gemäss einer besonders bevorzugten Ausführungsform ist das Peroxid aus der Gruppe ausgewählt, die aus Cumol-hydroperoxid, 1,1,3,3-Tetramethylbutyl-hydroperoxid, t-Butyl-hydroperoxid, t-Amylhydroperoxid, Di-Isopropylbenzen-mono-hydroperoxid und einer Mischung aus mindestens zwei davon besteht. Bei dem Barbitursäurederivat handelt es sich vorzugsweise um ein Barbitursäurederivat ausgewählt aus der Gruppe bestehend aus 1-monosubstituierten Barbituraten, 5-monosubstituierten Barbituraten, 1,5-disubstituierten Barbituraten und 1,3,5-trisubstituierten Barbituraten. Gemäss einer besonderen Ausgestaltung der erfindungsgemässen Paste ist das Barbitursäurederivat ausgewählt aus der Gruppe bestehend aus 1,5-disubstituierten Barbituraten und 1,3,5-trisubstituierten Barbituraten. Es können vorzugsweise 1,5-disubstituierte Thiobarbiturate oder 1,3,5-trisubstituierte Thiobarbiturate eingesetzt werden. Gemäss einer bevorzugten Ausführungsform weisen die Substituenten jeweils eine Länge von 1 bis 10 Kohlenstoffatomen auf. Gemäss einer besonders bevorzugten Ausführungsform ist das Barbitursäurederivat aus der Gruppe aus 1-Cyclohexyl-5-ethyl-barbitursäure, 1-Phenyl-5-ethyl-barbitursäure und 1,3,5-Trimethyl-barbitursäure ausgewählt.

Als Polymerisationsbeschleuniger sind Schwermetallverbindungen, die aus der Gruppe bestehend aus Schwermetallsalzen und Schwermetallkomplexen ausgewählt sind, bevorzugt. Erfindungsgemäss bevorzugte Schwermetallverbindungen sind ausgewählt aus der Gruppe bestehend aus Kupfer(II)-hydroxid, Kupfer(II)methacrylat, Kupfer(II)acetylacetonat, Kupfer(II)-2-ethyl-hexanoat, Cobalt(II)-hydroxid, Cobalt(II)-2-ethyl-hexanoat, basischem Kupfer(II)-carbonat, Eisen(II)-2-ethyl-hexanoat, Eisen(III)-2-ethyl-hexanoat und einer Mischung aus mindestens zwei davon.

Gemäss einer anderen Ausgestaltungsform kann der Knochenzement oder mindestens eine Paste, Flüssigkeit oder Pulverkomponente einen Polymerisationsbeschleuniger umfassen, der ausgewählt aus der Gruppe bestehend aus N,N-Dimethyl-p-toluidin, N,N-Bis-hydroxyethyl-p-toluidin, N,N-Dimethyl-anilin, Trioctylmethylammoniumchlorid, Tetrabutylammoniumchlorid, Lithiumchlorid, Saccharin, 1,8-Diazabicyclo[5.4.0]undec-7-en und 1,5-Diazabicyclo(4.3.0)non-5-en, Phthalimid, Maleimid, Succinimid, Pyromelithsäuredümid und einer Mischung aus mindestens zwei davon.

Eine weitere vorteilhafte Ausgestaltung der Erfindung umfasst eine Verwendung von Kombinationen aus Schwermetallsalzen und mindestens einem Vertreter aus der Gruppe umfassend N,N-Dimethyl-p-toluidin, N,N-Bis-hydroxyethyl-p-toluidin, N,N-Dimethyl-anilin, Trioctylmethylammoniumchlorid, Tetrabutylammoniumchlorid, Lithiumchlorid, Saccharin, 1,8-Diazabicyclo[5.4.0]undec-7-en und 1,5-Diazabicyclo(4.3.0)non-5-en, Phthalimid, Maleimid, Succinimid und Pyromelithsäurediimid als Polymerisationsbeschleuniger. Dabei werden Zweifachkombinationen und auch Dreifachkombinationen von unterschiedlichen Polymerisationsbeschleunigern im Rahmen der Erfindung offenbart.

Eine vorteilhafte Ausgestaltung der Erfindung besteht darin, dass gegebenenfalls wenigstens ein Co-Polymerisationsbeschleuniger in der erfindungsgemässen Zusammensetzung oder in einer der Pasten A, B oder der Flüssigkeit B oder Pulverkomponente A enthalten ist, wobei als Co-Polymerisationsbeschleuniger tertiäre Amine und Amidine bevorzugt sind, und wobei ganz besonders N,N-Dimethyl-p-toluidin, N,N-Bis-hydroxyethyl-p-toluidin, N,N-Dimethylanilin, 1,8-Diazabicyclo[5.4.0-]undec-7-en und 1,5-Diazabicyclo(4.3.0)-non-5-en als Co-Akzeleratoren bevorzugt sind.

Der erfindungsgemässe Knochenzement, insbesondere in Form einer Paste kann den Polymerisationsinitiator, den Polymerisationsbeschleuniger, den Co-Polymerisationsbeschleuniger oder den Polymerisationsinitiator, den Polymerisationsbeschleuniger und den Co-Polymerisationsbeschleuniger in einer (Gesamt)Menge von bis zu 10 Gew.-%, bezogen auf das Gesamtgewicht des Knochenzementes oder jeweils unabhängig bezogen auf das Gesamtgewicht einer der Pasten A, B, Flüssigkeit B oder Pulverkomponente A, beinhalten.

Der erfindungsgemässe Knochenzement, insbesondere in Form einer Paste oder die Pasten A, B oder Flüssigkeit B als auch die Pulverkomponente A können neben den vorstehend genannten Komponenten weitere Bestandteile beinhalten.

Gemäss einer bevorzugten Ausführungsform des erfindungsgemässen Knochenzementes oder einer der Pasten A, B, Flüssigkeit B oder Pulverkomponente A können diese jeweils unabhängig voneinander mindestens einen Röntgenopaker beinhalten. Beim Röntgenopaker kann es sich um einen fachüblichen Röntgenopaker handeln. Geeignete Röntgenopaker können in dem radikalisch polymerisierbaren Monomer löslich oder unlöslich sein. Der Röntgenopaker ist vorzugsweise aus der Gruppe ausgewählt, die aus Metalloxiden (wie zum Beispiel Zirkoniumdioxid), Bariumsulfat, toxikologisch unbedenklichen Schwermetallpartikeln (wie zum Beispiel Tantal), Ferrit, Magnetit (ggf. auch supramagnetisches Magnetit) und biokompatiblen Kalziumsalzen besteht. Diese Röntgenopaker weisen vorzugsweise einen mittleren Teilchendurchmesser im Bereich von 10 nm bis 500 µm auf. Als Röntgenopaker kommen ferner auch Ester der 3,5-Bis(acetamido)-2,4,6-triiodbenzoesäure, Gadolinium-Verbindungen, wie Gadolinium-Chelat mit den Estern der 1,4,7,10-Tetraazacyclododecan-1,4,7,10-tetraessigsäure (DOTA), in Betracht. Die Konzentrationen an Röntgenopaker, insbesondere die Konzentration an Zirkoniumdioxid, in dem Knochenzement oder einer der Pasten A, B, Flüssigkeit B oder Pulverkomponente A können jeweils voneinander unabhängig beispielsweise in einem Bereich von 3 bis 30 Gew.-% in Bezug auf die jeweilige Gesamtzusammensetzung liegen. Röntgenopaker gelten vorliegend nicht als Füllstoffe.

Gemäss einer weiteren bevorzugten Ausführungsform kann der erfindungsgemässe Knochenzement oder mindestens eine der Pasten A, B, die Flüssigkeit B oder die Pulverkomponente A mindestens einen Stabilisator beinhalten. Der Stabilisator soll geeignet sein, eine Spontanpolymerisation der in der Paste enthaltenen radikalisch polymerisierbaren Monomere zu verhindern. Ferner soll der Stabilisator keine störenden Wechselwirkungen mit den anderen in der erfindungsgemässen Paste enthaltenen Bestandteilen zeigen. Derartige Stabilisatoren sind aus dem Stand der Technik bekannt. Gemäss einer bevorzugten Ausführungsform handelt es sich bei dem Stabilisator um 2,6-Di-tert-butyl-4-methylphenol und/oder 2,6-Di-tert-butyl-phenol.

Die Erfindung wird durch die nachstehenden Beispiele erläutert, ohne jedoch die Erfindung auf diese Beispiele zu beschränken.

### Ausführungsbeispiele

### Herstellung von umhüllten Amphotericin B-Partikeln

### Partikel für Beispiel 1

Es wurden 2,000 g N-Methylglcamin in ein Rollrandglas eingewogen. Unter Rühren wurde das N-Methylglucamin bei einer Temperatur von 140 °C aufgeschmolzen. Nach vollständigem Aufschmelzen des N-Methylglucamins wurden 2,000 g Palmitinsäure zugesetzt und 10 Minuten weiter bei 140 °C gerührt. Danach wurden 0,400 g Amphotericin unter Rühren zugesetzt, bis die Amphoterin B-Partikel vollständig in der Schmelze dispergiert sind. Danach wurde sofort auf Raumtemperatur abgekühlt. Die Schmelze erstarrte. Danach wurde die Schmelze mit einem Mörser zu Pulver aufgemahlen.

### Partikel für Beispiel 2

Es wurden 1,000 g N-Methylglcamin in ein Rollrandglas eingewogen. Unter Rühren wurde das N-Methylglucamin bei einer Temperatur von 140 °C aufgeschmolzen. Nach vollständigem Aufschmelzen des N-Methylglucamins wurden 1,000 g Palmitinsäure zugesetzt und 10 Minuten weiter bei 140 °C gerührt. Danach wurden 0,400 g Amphotericin unter Rühren zugesetzt, bis die Amphoterin B-Partikel vollständig in der Schmelze dispergiert sind. Danach wurde sofort auf Raumtemperatur abgekühlt. Die Schmelze erstarrte. Danach wurde die Schmelze mit einem Mörser zu Pulver aufgemahlen.

### Partikel für Beispiel 3

Es wurden 0,700 g N-Methylglcamin in ein Rollrandglas eingewogen. Unter Rühren wurde das N-Methylglucamin bei einer Temperatur von 140 °C aufgeschmolzen. Nach vollständigem Aufschmelzen des N-Methylglucamins wurden 0,700 g Palmitinsäure zugesetzt und 10 Minuten weiter bei 140 °C gerührt. Danach wurden 0,400 g Amphotericin unter Rühren zugesetzt, bis die Amphoterin B-Partikel vollständig in der Schmelze dispergiert sind. Danach wurde sofort auf Raumtemperatur abgekühlt. Die Schmelze erstarrte. Danach wurde die Schmelze mit einem Mörser zu Pulver aufgemahlen.

Mit den zuvor hergestellten Amphotericin B-Partikeln wurden die Zementpulver der Beispiele 1-3 hergestellt. Das Zementpulver des Beispiels 4 war als Kontrolle aus Palacos R und reinem Additiv zusammengesetzt. Das Zementpulver des Beispiels 5 bestand aus reinem Palacos R-Zementpulver.

**Zusammensetzung der Zementpulver der Beispiele 1-5**

| Beispiel | Zusammensetzung der Zementpulver | | | |
|---|---|---|---|---|
| | Palacos R [g] | Partikel | | |
| | | Amphotericin B [mg] | 1-N-Methyl-D-glucamin [mg] | Palmitinsäure [mg] |
| 1 | 40,0 | 45 | 227 | 227 |
| 2 | 40,0 | 83 | 208 | 208 |
| 3 | 40,0 | 111 | 194 | 294 |
| 4 | 40,0 | - | 250 | 250 |
| 5 | 40,0 | - | - | - |

Für die nachfolgende Herstellung von Probekörpern wurde jeweils 40,5 g Zementpulver der Beispiele 1 bis 5 mit jeweils 20 ml Monomerflüssigkeit vermischt. Die Monomerflüssigkeit war jeweils aus 18,50 Methylmethacrylat, 0,38 g N,N-Dimethyl-p-toluidin, 0,002 g Hydrochinon und Spuren von Chlorophyllin (E241) zusammengesetzt. Nach der Vermischung der Zementpulver der Beispiele 1-4 mit jeweils 20 ml Monomerflüssigkeit entstand nach ca. 60 Sekunden ein plastisch verformbarer, grünlicher Zementteig, der zur Herstellung von Prüfkörpern verwendet wurde. Der Zementteig härtete nach ca. 4 Minuten aus.

Es wurden für die Bestimmung der Biegefestigkeit und des Biegemoduls gemäss ISO 5833 streifenförmige Prüfkörper mit den Abmessungen 3,3 mm x 10,0 mm x 75 mm hergestellt. Für die Bestimmung der Druckfestigkeit wurden zylinderförmige Probekörper mit einem Durchmesser von 6 mm und mit einer Höhe von 10 mm gefertigt. Die Prüfung der Biegefestigkeit, des Biegemoduls und der Druckfestigkeit gemäss der ISO 5833 erfolgte mit einer Zwick-Universalprüfmaschine Z010.

| Beispiel | Biegefestigkeit [MPa] | Biegemodul [MPa] | Druckfestigkeit [MPa] |
|---|---|---|---|
| 1 | 63,3 ± 1,7 | 2703 ± 70 | 80,9 ± 4,2 |
| 2 | 61,8 ± 1,3 | 2630 ± 122 | 85,5 ± 0,8 |
| 3 | 63,4 ± 0,9 | 2743 ± 84 | 86,5 ± 1,9 |
| 4 | 61,9 ± 1,1 | 2625 ± 50 | 86,6 ± 2,7 |
| 5 | 69,6 ± 1,3 | 2897 ± 117 | 91,9 ± 1,7 |

Die Norm ISO 5833 fordert für eine Biegefestigkeit grösser 50 MPa, ein Biegemodul grösser 1800 MPa und eine Druckfestigkeit von grösser 70 MPa. Die mit Amphotericin B und Additiv modifizierten Knochenzemente der Beispiele 1-3 erfüllen die Anforderungen der ISO 5833 hinsichtlich der Biegefestigkeit, des Biegemoduls und der Druckfestigkeit.

Zur Prüfung der antimykotischen Wirkung wurden mit den Zementen der Beispiele 1-5 zylinderförmige Prüfkörper mit einem Durchmesser von 20 mm und einer Höhe von 3 mm hergestellt. Die Prüfung der antimykotischen Wirkung wurde in Anlehnung an DIN 58940-3 und Pharm. Eur. Kapitel 2.7.2 vorgenommen. Als Prüfkeim diente *Candida albicans* ATCC 10231. Es wurden von jedem Beispiel drei Prüfkörper parallel auf ihre antimykotische Wirkung geprüft.

Die Hefen wurden in 2 Passagen auf Caseinpepton-Sojamehlpepton-Agar (TSA) für 40 bis 48 Stunden bei 35-37 °C angezogen. Anschließend wurden die Keime mit 5 ml 0,9 %-iger NaCl-Lösung abgeschwemmt und mit Hilfe von photometrischer Extinktionsmessungen bei 575 nm auf ca. 10⁶ KBE/ml eingestellt. 100 µl Keimsuspension wurden in je 20 ml auf 42-45 °C temperiert (TSA) (TSA, flüssig) gegeben, so dass sich eine Endkonzentration von ca. 10⁵ KBE pro Test ergab. Jede Testprobe wurde im Dreifachansatz untersucht. Die Platten wurden bei 35-37 °C für 48 Stunden inkubiert. Anschließend wurde der jeweilige Hemmhof (= keimfreie Zone) auf den Platten in Millimetern bestimmt.

| | Hemmhofdurchmesser [mm] | | |
|---|---|---|---|
| Beispiel | Prüfkörper 1 | Prüfkörper 2 | Prüfkörper 3 |
| 1 | 32 | 30 | 32 |
| 2 | 31 | 33 | 32 |
| 3 | 35 | 34 | 35 |
| 4 | - | - | - |
| 5 | - | - | - |

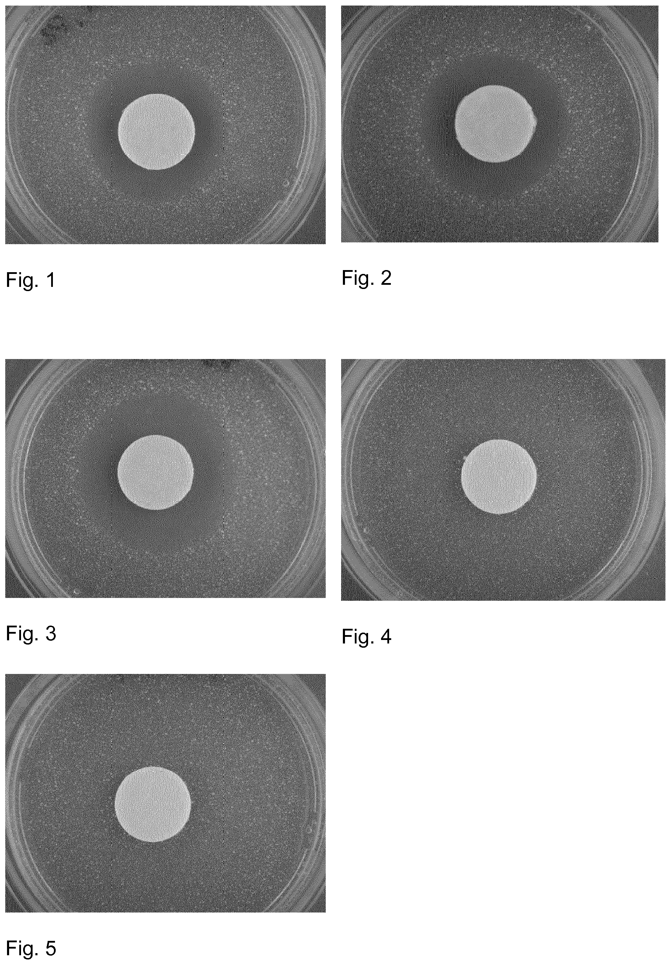

Die Figuren 1 bis 5 zeigen:
Fig. 1: Agarplatte mit Prüfkörper des Beispiels 1
Fig. 2: Agarplatte mit Prüfkörper des Beispiels 2
Fig. 3: Agarplatte mit Prüfkörper des Beispiels 3
Fig. 4: Agarplatte mit Prüfkörper des Beispiels 4
Fig. 5: Agarplatte mit Prüfkörper des Beispiels 5

Alle Prüfkörper der Beispiele 1-3 zeigten einen eindeutig erkennbaren Hemmhof. Die Prüfkörper des Beispiels 5 zeigten keine Hemmung des Wachstums der Hefe. Der Zement Palacos R besitzt offensichtlich keine antimykotische Wirkung. Ebenso zeigten die Prüfkörper des Beispiels 4 keine Hemmwirkung auf das Wachstum der Hefe. Das bedeutet, dass der Zement Palacos R zusammen mit dem Additiv ebenfalls keine antimykotische Wirkung besitzt. Der antimykotische Effekt der Prüfkörper der Beispiele 1-3 ist auf die Amphotericin B-Partikel zurück zu führen.

Es wurden analog zu den Amphotericin B-Partikeln der Beispiele 1-3 auch Amphotericn B-Partikel unter Verwendung der Kombination N-Methylglucamin mit Laurinsäure, N-Methylglucamin mit Myristinsäure und N-Methylglucamin mit Stearinsäure hergestellt. Diese Partikel zeigten ein vergleichbares Verhalten im Polymethylmethacrylat-Knochenzement wie die mit der Kombination N-Methylglucamin mit Palmitinsäure.

## Patentansprüche

1. Polymerisierbarer Knochenzement umfassend
(i) mindestens ein radikalisch polymerisierbares Monomer
(ii) mindestens ein in (i) lösliches organisches Polymer, und
(iii) mindestens einen Polymerisaitonsinitiator,
(iv) mindestens einen Röntgenopaker,
**dadurch gekennzeichnet, dass**
der Knochenzement mindestens ein Antimykotikum im Gemisch mit mindestens einem Amino-funktionellen Lösungsvermittler und mindestens einer Carbonsäure als Lösungsvermittler umfasst, wobei das Antimykotikum umhüllt ist von 1-Methylamino-1-desoxy-zuckeralkohol und mindestens einer Fettsäure.

2. Knochenzement nach Anspruch 1, **dadurch gekennzeichnet, dass** er zwei Komponenten A und B umfasst, wobei
(i) Komponente A als Paste vorliegt und umfasst
(a1) mindestens ein radikalisch polymerisierbares Monomer,
(a2) mindestens ein in (a1) lösliches organisches Polymer, und
(a3) mindestens einen Polymerisationsinitiator; und
Komponente B als Paste vorliegt und umfasst
(b1) mindestens ein radikalisch polymerisierbares Monomer,
(b2) mindestens ein in (i), (b1) lösliches organisches Polymer, und
(b3) mindestens einen Polymerisationsbeschleuniger, oder
(ii) Komponente A als Pulver vorliegt und umfasst
(a1) mindestens ein pulverförmiges Polyacrylat,
(a2) mindestens einen pulverförmigen Röntgenopaker, und
(a3) mindestens einen Polymerisationsinitiator; und
Komponente B als Flüssigkeit oder Paste vorliegt und umfasst
(b1) mindestens ein radikalisch polymerisierbares Monomer,
(b2) optional mindestens ein in (ii), (a1) lösliches organisches Polymer, und
(b3) mindestens einen Polymerisationsbeschleuniger; und,
wobei mindestens die Komponente A und/oder die Komponente B als (a4) und/oder (b4) mindestens ein Antimykotikum mit mindestens einem Lösungsvermittler aufweisen.

3. Knochenzement nach Anspruch 1 oder 2, **dadurch gekennzeichnet, dass** das Antimykotikum Amphotericin B ist.

4. Knochenzement nach einem der Ansprüche 1 bis 3, **dadurch gekennzeichnet, dass** das Antimykotikum im Gemisch mit mindestens einer Carbonsäure als Lösungsvermittler vorliegt, wobei der Lösungsvermittler umfasst Carbonsäuren mit Alkyl-und/oder Aryl-Resten, wobei die Carbonsäure mindestens 8 C-Atome aufweist.

5. Knochenzement nach Anspruch 4, **dadurch gekennzeichnet, dass** die Carbonsäure umfasst Palmitinsäure, Laurinsäure, Myristinsäure, Stearinsäure, Nonansäure, Decansäure, Undecansäure, Tridecansäure, Pentadecansäure, Heptadecansäure, Octadecansäure, Nonadecansäure, Eicosan-/Icosansäure, Heneicosansäure, Docosansäure, Tetracosansäure, Hexacosansäure

6. Knochenzement nach einem der Ansprüche 1 bis 5, **dadurch gekennzeichnet, dass** das Antimykotikum Amphotericin B ist und im Gemisch von Lösungsvermittlern umfassend mindestens ein Amino-funktionelles Polysaccharid, Aminocholesterol, Amino-Polyether oder Derivate davon und mindestens einer Carbonsäure vorliegt.

7. Knochenzement nach einem der Ansprüche 1 bis 6, **dadurch gekennzeichnet, dass** das Antimykotikum, vorzugsweise Amphotericin B, umhüllt ist von 1-Methylamino-1-desoxy-zuckeralkohol und mindestens einer Fettsäure, besonders bevorzugt ist Amphotericin B, umhüllt von N-Methyl-D-Glucamin (Meglumin) und mindestens einer Fettsäure.

8. Knochenzement nach einem der Ansprüche 1 bis 7, **dadurch gekennzeichnet, dass** das (i) Monomer ausgewählt ist aus mindestens einem Alkyl-2-acrylsäurealkylester, Aryl-2-acrylsäurealkylester, Arylalkyl-2-acrylsäurealkylester, jeweils unabhängig mit 1 bis 20 C-Atomen in der Alkyl-Gruppe, jeweils unabhängig mit 6 bis 14 C-Atomen in der Aryl-Gruppe, jeweils unabhängig mit 6 bis 14 C-Atomen in der Arylalkyl-Gruppe und jeweils unabhängig mit 1 bis 10 C-Atomen in der Alkylester-Gruppe oder einer Mischung umfassend mindestens zwei der genannten Monomere, und/oder das (ii) organische Polymer ausgewählt ist aus mindestens einem Poly(alkyl-2-acrylsäure-alkylester), Poly(aryl-2-acrylsäurealkylester), Poly(arylalkyl-2-acrylsäurealkylester), jeweils unabhängig mit 1 bis 20 C-Atomen in der Alkyl-Gruppe, jeweils unabhängig mit 6 bis 14 C-Atomen in der Aryl-Gruppe, jeweils unabhängig mit 6 bis 14 C-Atomen in der Arylalkyl-Gruppe und jeweils unabhängig mit 1 bis 10 C-Atomen in der Alkylester-Gruppe oder einer Mischung umfassend mindestens zwei der genannten Polymere.

9. Verfahren zur Herstellung eines Zwischenproduktes, indem mindestens zwei Lösungsvermittler umfassend einen Amino-funktionellen Lösungsvermittler und mindestens eine Carbonsäure oder ein Gemisch an Lösungsvermittlern umfassend einen Amino-funktionellen Lösungsvermittler und mindestens eine Carbonsäure und ein Antimykotikum gemischt werden, **dadurch gekennzeichnet, dass**
(i)
a) mindestens ein Amino-funktioneller Lösungsvermittler und mindestens eine Carbonsäure mit Alkyl- und/oder Aryl-Resten, wobei die Carbonsäure mindestens 8 C-Atome aufweist, geschmolzen werden und zu dem geschmolzenen Gemisch als Antimykotikum Amphotericin B zugefügt wird,
b) das geschmolzene Gemisch umfassend Amphotericin B abgekühlt wird und optional durch Einwirken mechanischer Energie zerkleinert wird, wobei es sich bei dem Amino-funktionellen Lösungsvermittler um 1-Methylamino-1-desoxy-zuckeralkohol handelt.

10. Zwischenprodukt erhältlich nach einem Verfahren nach Anspruch 9.

11. Zusammensetzung zur Verwendung zur Behandlung und/oder Prävention von Mykosen, **dadurch gekennzeichnet, dass** die Zusammensetzung umfasst,
(i) mindestens ein radikalisch polymerisierbares Monomer
(ii) mindestens ein in (i) lösliches organisches Polymer, und
(iii) mindestens einen Polymerisationsinitiator, und mindestens ein Antimykotikum,
(iv) Röntgenopaker,
(v) optional mindestens ein Polymerisationsbeschleuniger, umfassend mindestens ein Antimykotikum im Gemisch mit mindestens zwei Lösungsvermittlern umfassend mindestens einen Amino-funktionellen Lösungsvermittler und mindestens eine Carbonsäure, wobei das Antimykotikum umhüllt ist von 1-Methylamino-1-desoxy-zuckeralkohol und mindestens einer Fettsäure.

12. Kit zur Herstellung von polymerisierbarem Knochenzement, **dadurch gekennzeichnet,**
**dass** es die Komponenten A und B umfasst, wobei
(i) Komponente A als Paste vorliegt und umfasst
(a1) mindestens ein radikalisch polymerisierbares Monomer,
(a2) mindestens ein in (a1) lösliches organisches Polymer, und
(a3) mindestens einen Polymerisationsinitiator; und
Komponente B als Paste vorliegt und umfasst
(b1) mindestens ein radikalisch polymerisierbares Monomer,
(b2) mindestens ein in (i), (b1) lösliches organisches Polymer, und
(b3) mindestens einen Polymerisationsbeschleuniger; oder
(ii) Komponente A als Pulver vorliegt und umfasst
(a1) mindestens ein pulverförmiges Polyacrylat,
(a2) mindestens einen pulverförmigen Röntgenopaker, und
(a3) mindestens einen Polymerisationsinitiator; und
Komponente B als Flüssigkeit oder Paste vorliegt und umfasst
(b1) mindestens ein radikalisch polymerisierbares Monomer,
(b2) optional mindestens ein in (ii), (a1) lösliches organisches Polymer, und
(b3) mindestens einen Polymerisationsbeschleuniger und,
wobei mindestens die Komponente A und/oder die B als (a4) und/oder (b4) mindestens ein Antimykotikum und mindesten zwei Lösungsvermittler umfassend einen Amino-funktionellen Lösungsvermittler und mindestens eine Carbonsäure umfasst, , wobei das Antimykotikum umhüllt ist von 1-Methylamino-1-desoxy-zuckeralkohol und mindestens einer Fettsäure.

13. Polymerisierbarer Knochenzement erhältlich durch Vermischen der Komponenten A und B nach Anspruch 2 oder nach Anspruch 12.

14. Polymerisierter, gehärteter Knochenzement zur Behandlung und/oder Prävention von Mykosen erhältlich durch Vermischen der Komponenten A und B nach Anspruch 2 oder nach Anspruch 12, **dadurch gekennzeichnet, dass** das Antimykotikum in Gegenwart von Feuchte, Wasser, wässrigem Milieu, wie Körperflüssigkeiten, oder einer wässrigen Lösung, freigesetzt wird.

15. Formkörper erhältlich durch Formen und Polymerisation des Knochenzements nach Anspruch 13.

16. Chirurgisches Implantat zur Verwendung zur Behandlung und/oder Prävention einer Mykose enthaltend einen Knochenzement oder einen Formkörper nach einem der Ansprüche 13 bis 15, insbesondere als chirurgisches Implantat oder Teil eines Implantates, antimykotisches Implantat, Revisionsimplantat, Schraube, Nagel, chirurgische Platte, zur mechanischen Fixierung primärer Totalgelenkendoprothesen, zur mechanischen Fixierung von Revisions-Totalgelenkendoprothesen, zur Augmentation von osteoporotischem Knochengewebe und ganz besonders bevorzugt für die Vertebroplastie, Kyptroplastie und die Augmentation von Bohrlöchern in osteoporotischem Knochengewebe, zur Auffüllung von Knochenkavitäten, zur Femuroplastie, zur Herstellung von Spacern, zur mechanischen Fixierung von Gelenkprothesen, zur Abdeckung von Schädeldefekten oder zur Herstellung von Trägermaterialien für die lokale Antibiotika-Therapie oder als Trägermaterial für eine lokale Freisetzung von pharmazeutisch wirksamen Substanzen.

## Claims

1. Polymerisable bone cement, comprising
(i) at least one monomer for radical polymerisation;
(ii) at least one organic polymer that is soluble in (i); and
(iii) at least one polymerisation initiator;
(iv) at least one radiopaquer;
**characterised in that**
the bone cement comprises at least one antifungal agent in a mixture with at least one amino-functional solubilising agent and at least one carboxylic acid as solubilising agent, whereby the antifungal agent is enveloped by 1-methylamino-1-deoxy-sugar alcohol and at least one fatty acid.

2. Bone cement according to claim 1, **characterised in that** it comprises two components A and B, whereby
(i) component A is present as a paste and comprises
(a1) at least one monomer for radical polymerisation;
(a2) at least one organic polymer that is soluble in (a1); and
(a3) at least one polymerisation initiator; and
component B is present as a paste and comprises
(b1) at least one monomer for radical polymerisation;
(b2) at least one organic polymer that is soluble in (i), (b1); and
(b3) at least one polymerisation accelerator; or
(ii) component A is present as a powder and comprises
(a1) at least one powdered polyacrylate;
(a2) at least one powdered radiopaquer; and
(a3) at least one polymerisation initiator; and
component B is present as a liquid or a paste and comprises
(b1) at least one monomer for radical polymerisation;
(b2) optionally, at least one organic polymer that is soluble in (ii), (a1); and
(b3) at least one polymerisation accelerator; and,
whereby at least component A and/or component B comprise, as (a4) and/or (b4), at least one antifungal agent with at least one solubilising agent.

3. Bone cement according to claim 1 or 2, **characterised in that** the antifungal agent is amphotericin B.

4. Bone cement according to any one of the claims 1 to 3, **characterised in that** the antifungal agent is present in a mixture containing at least one carboxylic acid as solubilising agent, whereby the solubilising agent comprises carboxylic acids with alkyl and/or aryl residues, whereby the carboxylic acid comprises at least 8 C-atoms.

5. Bone cement according to claim 4, **characterised in that** the carboxylic acid comprises palmitic acid, lauric acid, myristic acid, stearic acid, nonanoic acid, decanoic acid, undecanoic acid, tridecanoic acid, pentadecanoic acid, heptadecanoic acid, octadecanoic acid, nonadecanoic acid, eicosanoic/icosanoic acid,heneicosanoic acid, docosanoic acid, tetracosanoic acid, hexacosanoic acid.

6. Bone cement according to any one of the claims 1 to 5, **characterised in that** the antifungal agent is amphotericin B and is present in a mixture containing solubilising agents comprising at least one amino-functional polysaccharide, amino cholesterol, amino polyether or derivatives thereof, and at least one carboxylic acid.

7. Bone cement according to any one of the claims 1 to 6, **characterised in that** the antifungal agent, preferably amphotericin B, is enveloped by 1-methylamino-1-deoxysugar alcohol and at least one fatty acid, particularly preferably is amphotericin B enveloped by N-methyl-D-glucamine (meglumine) and at least one fatty acid.

8. Bone cement according to any one of the claims 1 to 7, **characterised in that** the (i) monomer is selected from at least one alkyl-2-acrylic acid alkyl ester, aryl-2-acrylic acid alkyl ester, arylalkyl-2-acrylic acid alkyl ester, each independently having 1 to 20 C-atoms in the alkyl group, each independently having 6 to 14 C-atoms in the aryl group, each independently having 6 to 14 C-atoms in the arylalkyl group, and each independently having 1 to 10 C-atoms in the alkyl ester group or a mixture comprising at least two of the aforementioned monomers, and/or **in that** the (ii) organic polymer is selected from at least one poly(alkyl-2-acrylic acid alkyl ester), poly(aryl-2-acrylic acid alkyl ester), poly(arylalkyl-2-acrylic acid alkyl ester), each independently having 1 to 20 C-atoms in the alkyl group, each independently having 6 to 14 C-atoms in the aryl group, each independently having 6 to 14 C-atoms in the arylalkyl group, and each independently having 1 to 10 C-atoms in the alkyl ester group or a mixture comprising at least two of the aforementioned polymers.

9. Method for the production of an intermediary product through the mixing of at least two solubilising agents comprising an amino-functional solubilising agent and at least one carboxylic acid or a mixture of solubilising agents comprising an amino functional solubilising agent and at least one carboxylic acid and an antifungal agent, **characterised in that**
(i)
a) at least one amino-functional solubilising agent and at least one carboxylic acid with alkyl and/or aryl residues, whereby the carboxylic acid comprises at least 8 C-atoms, are melted and amphotericin B is added to the melted mixture as an antifungal agent,
b) the molten mixture comprising amphotericin B is cooled down and, optionally, disintegrated through the action of mechanical energy, whereby the amino-functional solubilising agent is 1-methylamino-1-deoxysugar alcohol.

10. Intermediary product obtainable according to a method according to claim 9.

11. Composition for use for the treatment and/or prevention of mycoses, **characterised in that** the composition comprises
(i) at least one monomer for radical polymerisation;
(ii) at least one organic polymer that is soluble in (i); and
(iii) at least one polymerisation initiator; and at least one antifungal agent;
(iv) radiopaquer;
(v) optionally, at least one polymerisation accelerator;
comprising at least one antifungal agent in a mixture containing at least two solubilising agents comprising at least one amino-functional solubilising agent and at least one carboxylic acid, whereby the antifungal agent is enveloped by 1-methylamino-1-deoxy-sugar alcohol and at least one fatty acid.

12. Kit for the production of polymerisable bone cement, **characterised in that** it comprises the components A and B, whereby
(i) component A is present as a paste and comprises
(a1) at least one monomer for radical polymerisation;
(a2) at least one organic polymer that is soluble in (a1); and
(a3) at least one polymerisation initiator; and
component B is present as a paste and comprises
(b1) at least one monomer for radical polymerisation;
(b2) at least one organic polymer that is soluble in (i), (b1); and
(b3) at least one polymerisation accelerator; or
(ii) component A is present as a powder and comprises
(a1) at least one powdered polyacrylate;
(a2) at least one powdered radiopaquer; and
(a3) at least one polymerisation initiator; and
component B is present as a liquid or a paste and comprises
(b1) at least one monomer for radical polymerisation;
(b2) optionally, at least one organic polymer that is soluble in (ii), (a1); and
(b3) at least one polymerisation accelerator; and,
whereby at least component A and/or component B comprise, as (a4) and/or (b4), at least one antifungal agent and at least two solubilising agents comprising an amino-functional solubilising agent and at least one carboxylic acid, whereby the antifungal agent is enveloped by 1-methylamino-1-deoxy-sugar alcohol and at least one fatty acid.

13. Polymerisable bone cement obtainable by mixing component A and component B according to claim 2 or according to claim 12.

14. Polymerisable, cured bone cement for the treatment and/or prevention of mycoses, obtainable through the mixing of components A and B according to claim 2 or according to claim 12, **characterised in that** the antifungal agent is released in the presence of moisture, water, aqueous milieu, such as body fluids, or an aqueous solution.

15. Form bodies, obtainable by forming and polymerisation of the bone cement according to claim 13.

16. Surgical implant for use in a method for the treatment and/or prevention of mycoses, containing a bone cement or a form body according to any one of the claims 13 to 15, preferably as surgical implant or part of an implant, antifungal implant, revision implant, screw, nail, surgical plate, for mechanical fixation of primary total articular endoprostheses, for mechanical fixation of revision total articular endoprostheses, for augmentation of osteoporotic bone tissue and, particularly preferably, for vertebroplasty, kyphoplasty, and augmentation of drill holes in osteoporotic bone tissue, for the filling of bone cavities, for femuroplasty, for the manufacture of spacers, for mechanical fixation of articular endoprostheses, for coverage of skull defects or for the production of carrier materials for local antibiotics therapy or as carrier material for local release of pharmaceutically active substances.

## Revendications

1. Ciment osseux polymérisable comprenant
(i) au moins un monomère polymérisable par voie radicalaire,
(ii) au moins un polymère organique soluble dans (i), et
(iii) au moins un initiateur de polymérisation,
(iv) au moins un opacifiant radiographique,
**caractérisé en ce que**
le ciment osseux comprend au moins un antifongique dans le mélange avec au moins un agent de solubilisation amino-fonctionnel et au moins un acide carboxylique en tant qu'agent de solubilisation, dans lequel l'antifongique est enveloppé de 1-méthylamino-1-désoxy-polyol et d'au moins un acide gras.

2. Ciment osseux selon la revendication 1, **caractérisé en ce que** il comprend deux composants A et B, dans lequel
(i) le composant A est présent en tant que pâte et comprend
(a1) au moins un monomère polymérisable par voie radicalaire,
(a2) au moins un polymère organique soluble dans (a1), et
(a3) au moins un initiateur de polymérisation ; et
le composant B est présent en tant que pâte et comprend
(b1) au moins un monomère polymérisable par voie radicalaire,
(b2) au moins un polymère organique soluble dans (i), (b1), et
(b3) au moins un accélérateur de polymérisation ; ou
(ii) le composant A est présent en tant que pâte et comprend
(a1) au moins un polyacrylate pulvérulent,
(a2) au moins un opacifiant radiographique pulvérulent, et
(a3) au moins un initiateur de polymérisation ; et
le composant B est présent en tant que fluide ou pâte et comprend
(b1) au moins un monomère polymérisable par voie radicalaire,
(b2) en option au moins un polymère organique soluble dans (ii), (a1), et
(b3) au moins un accélérateur de polymérisation ; et
dans lequel au moins le composant A et/ou le composant B en tant que (a4) et/ou (b4) présentent au moins un antifongique avec au moins un agent de solubilisation.

3. Ciment osseux selon la revendication 1 ou 2, **caractérisé en ce que** l'antifongique est l'amphotéricine B.

4. Ciment osseux selon une des revendications 1 à 3, **caractérisé en ce que** l'antifongique est présent dans le mélange avec au moins un acide carboxylique en tant qu'agent de solubilisation, dans lequel l'agent de solubilisation comprend des acides carboxyliques avec des résidus alkyle et/ou aryle, dans lequel l'acide carboxylique présente au moins 8 atomes de C.

5. Ciment osseux selon la revendication 4, **caractérisé en ce que** l'acide carboxylique comprend l'acide palmitique, l'acide laurique, l'acide myristique, l'acide stéarique, l'acide nonanoïque, l'acide décanoïque, l'acide undécanoïque, l'acide tridécanoïque, l'acide pentadécanoïque, l'acide heptadécanoïque, l'acide octadécanoïque, l'acide nonadécanoïque, l'acide eicosanoïque/icosanoïque, l'acide heneicosanoïque, l'acide docosanoïque, l'acide tétracosanoïque, l'acide hexacosanoïque.

6. Ciment osseux selon une des revendications 1 à 5, **caractérisé en ce que** l'antifongique est l'amphotéricine B et est présent dans le mélange d'agents de solubilisation comprenant au moins un polysaccharide amino-fonctionnel, un aminocholestérol, un amino-polyéther ou des dérivés de ceux-ci et au moins un acide carboxylique.

7. Ciment osseux selon une des revendications 1 à 6, **caractérisé en ce que** l'antifongique, de préférence l'amphotéricine B, est enveloppé de 1-méthylamino-1-désoxy-polyol et d'au moins un acide gras, est de manière particulièrement préférée l'amphotéricine B, enveloppée de N-méthyl-D-glucamine (méglumine) et d'au moins un acide gras.

8. Ciment osseux selon une des revendications 1 à 7, **caractérisé en ce que** le (i) monomère est sélectionné parmi au moins un ester alkylique d'acide alkyle-2-acrylique, ester alkylique d'acide aryle-2-acrylique, ester alkylique d'acide arylalkyle-2-acrylique, à chaque fois indépendamment avec 1 à 20 atomes de C dans le groupe alkyle, à chaque fois indépendamment avec 6 à 14 atomes de C dans le groupe aryle, à chaque fois indépendamment avec 6 à 14 atomes de C dans le groupe arylalkyle et à chaque fois indépendamment avec 1 à 10 atomes de C dans le groupe ester alkylique ou un mélange comprenant au moins deux desdits monomères, et/ou le (ii) polymère organique est sélectionné parmi au moins un ester alkylique d'acide poly(alkyle-2-acrylique), ester alkylique d'acide poly(aryle-2-acrylique), ester alkylique d'acide poly(arylalkyle-2-acrylique), à chaque fois indépendamment avec 1 à 20 atomes de C dans le groupe alkyle, à chaque fois indépendamment avec 6 à 14 atomes de C dans le groupe aryle, à chaque fois indépendamment avec 6 à 14 atomes de C dans le groupe arylalkyle et à chaque fois indépendamment avec 1 à 10 atomes de C dans le groupe ester alkylique ou un mélange comprenant au moins deux desdits polymères.

9. Procédé de fabrication d'un produit intermédiaire, en ce qu'au moins deux agents de solubilisation comprenant un agent de solubilisation amino-fonctionnel et au moins un acide carboxylique ou un mélange d'agents de solubilisation comprenant un agent de solubilisation amino-fonctionnel et au moins un acide carboxylique et un antifongique sont mélangés, **caractérisé en ce que**
(i)
a) au moins un agent de solubilisation amino-fonctionnel et au moins un acide carboxylique avec des résidus alkyle et/ou aryle, dans lequel l'acide carboxylique présente au moins 8 atomes de C, sont fondus et de l'amphotéricine B est ajoutée au mélange fondu en tant qu'antifongique,
b) le mélange fondu comprenant l'amphotéricine B est refroidi et en option fractionné par action d'énergie mécanique, dans lequel il s'agit pour l'agent de solubilisation amino-fonctionnel de 1-méthylamino-1-désoxy-polyol.

10. Produit intermédiaire pouvant être obtenu selon un procédé selon la revendication 9.

11. Composition pour l'utilisation pour le traitement et/ou la prévention de mycoses, **caractérisée en ce que** la composition comprend
(i) au moins un monomère polymérisable par voie radicalaire,
(ii) au moins un polymère organique soluble dans (i), et
(iii) au moins un initiateur de polymérisation et au moins un antifongique,
(iv) un opacifiant radiographique,
(v) en option au moins un accélérateur de polymérisation,
comprenant au moins un antifongique dans le mélange avec au moins deux agents de solubilisation comprenant au moins un agent de solubilisation amino-fonctionnel et au moins un acide carboxylique,
dans laquelle l'antifongique est enveloppé de 1-méthylamino-1-désoxy-polyol et d'au moins un acide gras.

12. Kit de fabrication de ciment osseux polymérisable, **caractérisé en ce qu'**il comprend les composants A et B, dans lequel
(i) le composant A est présent en tant que pâte et comprend
(a1) au moins un monomère polymérisable par voie radicalaire,
(a2) au moins un polymère organique soluble dans (a1), et
(a3) au moins un initiateur de polymérisation ; et
le composant B est présent en tant que pâte et comprend
(b1) au moins un monomère polymérisable par voie radicalaire,
(b2) au moins un polymère organique soluble dans (i), (b1), et
(b3) au moins un accélérateur de polymérisation ; ou
(ii) le composant A est présent en tant que pâte et comprend
(a1) au moins un polyacrylate pulvérulent,
(a2) au moins un opacifiant radiographique pulvérulent, et
(a3) au moins un initiateur de polymérisation ; et
le composant B est présent en tant que fluide ou pâte et comprend
(b1) au moins un monomère polymérisable par voie radicalaire,
(b2) en option au moins un polymère organique soluble dans (ii), (a1), et
(b3) au moins un accélérateur de polymérisation, et
dans lequel au moins le composant A et/ou le B en tant que (a4) et/ou (b4) comprend au moins un antifongique et au moins deux agents de solubilisation comprenant un agent de solubilisation amino-fonctionnel et au moins un acide carboxylique,
dans lequel l'antifongique est enveloppé de 1-méthylamino-1-désoxy-polyol et d'au moins un acide gras.

13. Ciment osseux polymérisable pouvant être obtenu par mélange des composants A et B selon la revendication 2 ou selon la revendication 12.

14. Ciment osseux durci polymérisé pour le traitement et/ou la prévention de mycoses pouvant être obtenu par mélange des composants A et B selon la revendication 2 ou selon la revendication 12, **caractérisé en ce que** l'antifongique est libéré en présence d'humidité, d'eau, d'un milieu aqueux, tel que des fluides corporels, ou d'une solution aqueuse.

15. Corps moulé pouvant être obtenu par moulage et polymérisation du ciment osseux selon la revendication 13.

16. Implant chirurgical pour l'utilisation pour le traitement et/ou la prévention d'une mycose contenant un ciment osseux ou un corps moulé selon une des revendications 13 à 15, notamment en tant qu'implant chirurgical ou partie d'un implant, implant antifongique, implant de révision, vis, clou, plaque chirurgicale, pour la fixation mécanique d'endoprothèses articulaires totales primaires, pour la fixation mécanique d'endoprothèses articulaires totales de révision, pour l'augmentation de tissu osseux ostéoporotique et de manière tout particulièrement préférée pour la vertébroplastie, la kyphoplastie et l'augmentation de trous de trépan dans du tissu osseux ostéoporotique, pour le remplissage de cavités osseuses, pour la fémoroplastie, pour la fabrication d'écarteurs, pour la fixation mécanique de prothèses articulaires, pour le recouvrement de défauts crâniens ou pour la fabrication de matériaux de support pour l'antibiothérapie locale ou en tant que matériau de support pour une libération locale de substances pharmaceutiquement actives.
